# EUROPEAN PATENT APPLICATION

(11) **EP 2 647 329 A1**
(43) Date of publication of application: **09.10.2013**
(21) Application number: 11845834.8
(22) Date of filing: 22.11.2011
(51) Int. Cl.: A61B 1/00, A61B 5/07

(54) **RECEPTION DEVICE AND CAPSULE-TYPE ENDOSCOPE SYSTEM**

(30) Priority: 29.11.2010 JP 2010265759
(71) Applicant: Olympus Medical Systems Corp., Tokyo 151-0072 (JP)
(72) Inventor: KOIDE, Naoto, Tokyo 151-0072 (JP); HOMAN, Masatoshi, Tokyo 151-0072 (JP)
(74) Representative: Schmidt, Steffen
(86) International application number: PCT/JP2011/076932
(87) International publication number: WO 2012/073763

(57) **Abstract**

It is provided a receiving apparatus that can suppress power consumption to the minimum even when a plurality of active antennas including active circuits is used and it is also provided a capsule endoscope system. The receiving apparatus includes: a plurality of receiving antennas 41-48 in which active circuits 41b-48b are provided; and a selection controller 581 that performs control of selecting one receiving antenna that receives a radio signal transmitted from the outside from the plurality of receiving antennas 41-48 and supplying power only to the selected receiving antenna.

## Description

### Field

The present invention relates to a receiving apparatus that receives a radio signal transmitted from a capsule endoscope in a subject using an antenna outside the subject and a capsule endoscope system.

### Background

In the field of endoscopes, a capsule endoscope in which an imaging function and a radio communication function are incorporated in a capsule-shaped casing having a size such that the casing can be inserted to a digestive tract of a subject such as a patient has conventionally been proposed. The capsule endoscope moves inside the subject such as a digestive tract according to a peristaltic motion after the capsule endoscope is swallowed through the mouth of the subject. Then, the capsule endoscope sequentially images the inside of the subject to generate image data and sequentially transmits the image data over the radio.

The image data transmitted over the radio from the capsule endoscope in this way is received by a receiving apparatus via a receiving antenna provided outside the subject. The receiving apparatus stores the image data received via the receiving antenna in an internal memory.

By carrying the receiving apparatus having a radio communication function and a memory function, the subject can freely perform activities until the capsule endoscope is excreted after being swallowed. After an examination ends, an examiner such as a physician takes the image data stored in the memory of the receiving apparatus into an image display device and displays intra-subject images (for example, organ images) corresponding to the image data obtained by the capsule endoscope on a display of the image display device. The examiner observes the organ images displayed on the display and diagnoses the subject.

Typically, when the radio signal is received from the capsule endoscope, in the receiving apparatus, in which multiple receiving antennas are arranged to be distributed outside the subject, one receiving antenna having the strongest received strength is selected, and the radio signal is received by the selected receiving antenna. For example, a receiving apparatus is known in which multiple antennas arranged outside a subject are switched for reception, and the position of a capsule endoscope inside the subject which is the source of a radio signal is detected based on the strength of an electric field received by each of the antennas (see patent literature 1 and patent literature 2).

### Citation List

### Patent Literature

Patent Literature 1: Japanese Laid-open Patent Publication No. 2003-019111
Patent Literature 2: Japanese Laid-open Patent Publication No. 2007-124142

### Summary

### Technical Problem

The abovementioned receiving apparatus conventionally uses a passive antenna as a receiving antenna; however, it may use an active antenna including an active circuit as a receiving antenna in order to improve the transmission property.

However, if an active antenna is used instead of a passive antenna, it is necessary to always supply power to the active antenna to receive a radio signal from the capsule endoscope. Therefore, even if it is configured to receive a radio signal by an active antenna having the strongest received strength selected from a plurality of active antennas, because power is supplied to the unselected active antennas, power is consumed uselessly.

The present invention has been achieved to solve the above problems with the conventional technology and it is an object of the present invention to provide a receiving apparatus that can suppress power consumption to the minimum even when a plurality of active antennas including active circuits is used and to provide a capsule endoscope system.

### Solution to Problem

To solve the above problem and achieve the above object, a receiving apparatus according to the present invention includes a plurality of receiving antennas in which active circuits are provided; and a selection controller that performs control of selecting one receiving antenna that receives a radio signal transmitted from the outside from the plurality of receiving antennas.

Moreover, the above receiving apparatus according to the present invention further includes an abnormality detector that detects abnormality of the selected receiving antenna based on a voltage when power is supplied to the receiving antenna selected by the selection controller.

Moreover, in the above receiving apparatus according to the present invention, the abnormality is disconnection abnormality or short-circuit abnormality of the selected receiving antenna.

Moreover, the above receiving apparatus according to the present invention further includes an abnormality information adding unit that adds abnormality information indicating that abnormality has occurred in the receiving antenna to the radio signal received by the receiving antenna when the abnormality detector detects abnormality of the receiving antenna.

Moreover, the above receiving apparatus according to the present invention further includes a received electric-field strength detector that detects received electric-field strength of an electric field received by each of the plurality of receiving antennas. The selection controller selects the receiving antenna that receives the radio signal based on the received electric-field strength detected by the received electric-field strength detector.

Moreover, the above receiving apparatus according to the present invention further includes an antenna selection changeover switch unit that selects one antenna cable from a plurality of antenna cables respectively connected to the plurality of receiving antennas and performs switching of the connection; and an antenna power changeover selector that supplies power to the antenna cable selected from the plurality of antenna cables respectively connected to the plurality of receiving antennas. The selection controller drives the switching operation of the antenna selection changeover switch unit and the antenna power changeover selector in a synchronized manner.

Moreover, a capsule endoscope system according to the present invention includes a capsule endoscope that is inserted into a subject and transmits image data of the inside of the subject to the outside via a radio signal; and a receiving apparatus. The receiving apparatus includes a plurality of receiving antennas in which an active circuit is provided, and a selection controller that performs control of selecting one receiving antenna that receives a radio signal transmitted from the capsule endoscope from the plurality of receiving antennas and supplying power only to the selected receiving antenna.

Moreover, in the above capsule endoscope system according to the present invention, the receiving apparatus further includes an abnormality detector that detects abnormality of the selected receiving antenna based on a voltage when power is supplied to the receiving antenna selected by the selection controller.

Moreover, in the above capsule endoscope system according to the present invention, the abnormality is disconnection abnormality or short-circuit abnormality of the selected receiving antenna.

Moreover, in the above capsule endoscope system according to the present invention, the receiving apparatus further includes an abnormality information adding unit that adds abnormality information indicating that abnormality has occurred in the receiving antenna to the radio signal received by the receiving antenna when the abnormality detector detects abnormality of the receiving antenna.

Moreover, in the above capsule endoscope system according to the present invention, the receiving apparatus further includes a received electric-field strength detector that detects received electric-field strength of an electric field received by each of the plurality of receiving antennas, and the selection controller selects the receiving antenna that receives the radio signal based on the received electric-field strength detected by the received electric-field strength detector.

Moreover, in the above capsule endoscope system according to the present invention, the receiving apparatus further includes an antenna selection changeover switch unit that selects one antenna cable from a plurality of antenna cables respectively connected to the plurality of receiving antennas and performs switching of the connection; and an antenna power changeover selector that supplies power to the antenna cable selected from the plurality of antenna cables respectively connected to the plurality of receiving antennas. The selection controller drives the switching operation of the antenna selection changeover switch unit and the antenna power changeover selector in a synchronized manner.

### Advantageous Effects of Invention

According to the present invention, control is performed such that any one of receiving antenna is selected from a plurality of receiving antennas in which active circuits are provided as a receiving antenna that receives a radio signal transmitted from the outside and that power is supplied to only the selected receiving antenna. As a result, it is possible to suppress power consumption to the minimum even when a plurality of active antennas including active circuits is used.

### Brief Description of Drawings

[Fig. 1] FIG. 1 is a schematic diagram illustrating a schematic configuration of a capsule endoscope system using a receiving apparatus according to an exemplary embodiment of the invention.
[FIG. 2] FIG. 2 is a block diagram illustrating a schematic configuration of the receiving apparatus according to the exemplary embodiment of the invention.
[FIG. 3] FIG. 3 is a diagram illustrating the connection relation between an antenna power changeover selector and a receiving antenna of the receiving apparatus according to the exemplary embodiment of the invention.
[FIG. 4] FIG. 4 is a timing chart illustrating the switching timing of the receiving antennas of the receiving apparatus according to the exemplary embodiment of the invention.
[FIG. 5] FIG. 5 is a schematic diagram illustrating a schematic configuration of a capsule endoscope system using a receiving apparatus according to a modification example of the exemplary embodiment of the invention.

### Description of Embodiments

Hereinafter, a receiving apparatus and a capsule endoscope system according to an exemplary embodiment of the invention will be described with reference to the drawings. In the following description, a capsule endoscope system including a capsule endoscope that is inserted into the body of a subject so as to capture an in-vivo image of the subject is illustrated as an example of the receiving apparatus and the capsule endoscope system according to the invention. However, the invention is not limited to this exemplary embodiment.

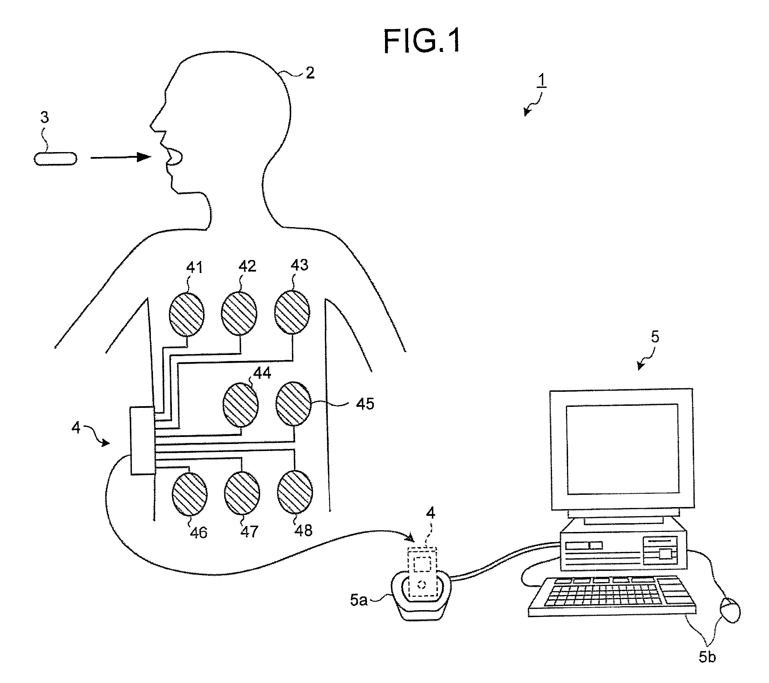

As illustrated in FIG. 1, a capsule endoscope system 1 includes a capsule endoscope 3 that captures an in-vivo image of a subject 2, a receiving apparatus 4 that receives a radio signal transmitted over the radio by the capsule endoscope 2 that is inserted into the subject 2, and an image display device 5 that displays an image corresponding to image data of the inside of the subject 2 imaged by the capsule endoscope 3.

The capsule endoscope 3 includes an imaging function of imaging the inside of the subject 2 and a radio communication function of transmitting image data obtained by imaging the inside of the subject 2 to the receiving apparatus 4. The capsule endoscope 3 is swallowed into the subject 2 to pass through the esophagus of the subject 2 and move in the body cavity according to the peristaltic motion of the digestive tract. The capsule endoscope 3 sequentially images the body cavity of the subject 2 at very small time intervals (for example, every 0.5 second) while moving in the body cavity, generates image data of the imaged inside of the subject 2, and sequentially transmits the image data to the receiving apparatus 4. In this case, the capsule endoscope 3 generates a transmission signal including the image data and transmits a radio signal obtained by modulating the generated transmission signal to the receiving apparatus 4 over the radio.

The receiving apparatus 4 includes multiple receiving antennas 41 to 48. The receiving apparatus 4 receives the radio signal transmitted over the radio from the capsule endoscope 3 via the respective receiving antennas 41 to 48. The receiving apparatus 4 detects the received electric-field strength of the radio signal received from the capsule endoscope 3 for each of the respective receiving antennas 41 to 48 and acquires the image data of the inside of the subject 2 based on the received radio signal. The receiving apparatus 4 stores information on the received electric-field strength of the respective receiving antennas 41 to 48 and time information representing time in memory in correlation with the received image data.

The receiving apparatus 4 is carried by the subject 2 during the period when the capsule endoscope 3 performs imaging, for example, until the capsule endoscope 3 passes through a digestive tract and is excreted from the subject 2 after being inserted through the mouth of the subject 2. After the examination of the capsule endoscope 3 ends, the receiving apparatus 4 is separated from the subject 2 and is connected to the image display device 5 in order to transmit information such as the image data received from the capsule endoscope 3.

The respective receiving antennas 41 to 48 are arranged at predetermined positions on the outer surface of the subject 2, for example, at positions corresponding to the respective internal organs of the subject 2 which is the passage of the capsule endoscope 3. The arrangement of the receiving antennas 41 to 48 may be changed optionally according to the purpose of examination or diagnosis. Further, the number of receiving antennas is not intended to be limited to 8 but may be smaller or greater than 8.

The image display device 5 is configured using a workstation or a personal computer that includes a display unit such as a liquid crystal display. The image display device 5 displays an image corresponding to the image data of the inside of the subject 2 acquired via the receiving apparatus 4. A cradle 5a for reading image data from the memory of the receiving apparatus 4 and an operation input device 5b such as a keyboard or a mouse are connected to the image display device 5. The cradle 5a acquires the image data and related information such as the received strength information, the time information, and the identification information of the capsule endoscope 3, correlated with the image data from the memory of the receiving apparatus 4 and transmits the acquired various types of information to the image display device 5 when the receiving apparatus 4 is attached thereto. The operation input device 5b receives the input from the user. In this way, the user observes biological portions inside the subject 2 such as, for example, the esophagus, the stomach, the small intestine, or the large intestine and diagnoses the subject 2 while operating the operation input device 5b and viewing the images of the inside of the subject 2 displayed sequentially by the image display device 5.

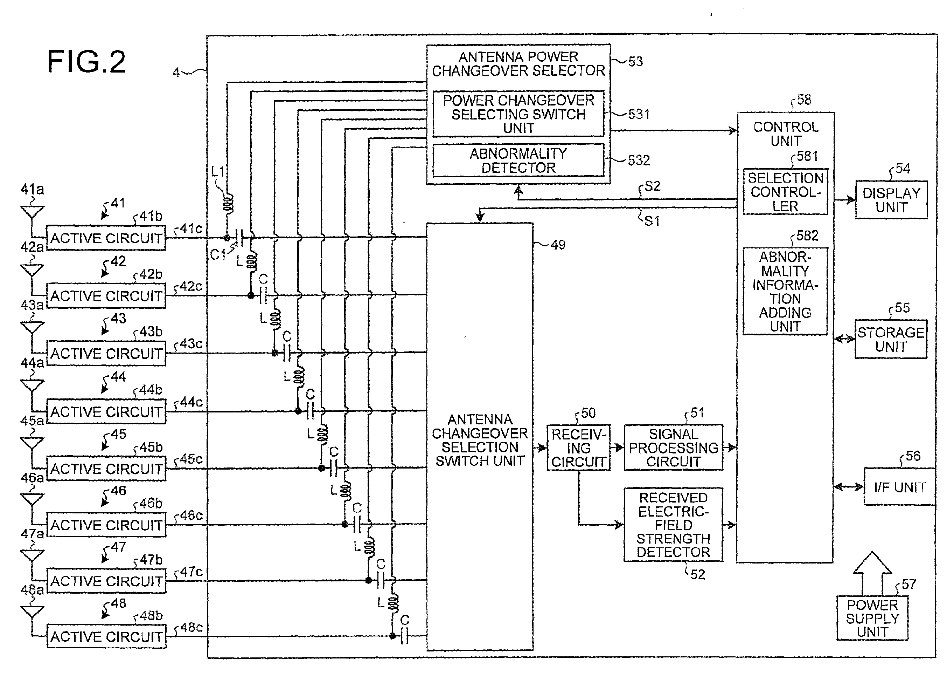

Next, the configuration of the receiving apparatus illustrated in FIG. 1 will be described in detail. FIG. 2 is a block diagram illustrating the configuration of the receiving apparatus 4 illustrated in FIG. 1.

As illustrated in FIG. 2, the receiving apparatus 4 includes the respective receiving antennas 41 to 48 described above, an antenna changeover selection switch unit 49 that selectively switches the receiving antennas 41 to 48, a receiving circuit 50 that performs processing such as demodulation on the radio signal received via any one of the respective receiving antennas 41 to 48 selected by the antenna changeover selection switch unit 49, a signal processing circuit 51 that performs signal processing of extracting image data from the radio signal output from the receiving circuit 50, a received electric-field strength detector 52 that detects received electric-field strength based on the strength of the radio signal output from the receiving circuit 50, an antenna power changeover selector 53 that selectively switches the receiving antennas 41 to 48 so as to supply power to any one of the receiving antennas 41 to 48, a display unit 54 that displays an image corresponding to the image data received from the capsule endoscope 3, a storage unit 55 that stores various types of information including the image data received from the capsule endoscope 3, an I/F unit 56 that performs bidirectional communication with the image display device 5 via the cradle 5a, a power supply unit 57 that supplies power to the respective units of the receiving apparatus 4, and a control unit 58 that controls the operation of the receiving apparatus 4.

The receiving antenna 41 includes an antenna unit 41a, an active circuit 41b, and an antenna cable 41c. The antenna unit 41a is configured using an open-type antenna, for example, and receives the radio signal transmitted from the capsule endoscope 3. The active circuit 41b is connected to the antenna unit 41a so as to perform impedance matching of the antenna unit 41a and amplify or attenuate the radio signal. The antenna cable 41c is configured using a coaxial cable and has one end connected to the active circuit 41a and the other end electrically connected to the antenna changeover selection switch unit 49 and the antenna power changeover selector 53 of the receiving apparatus 4. The antenna cable 41c transmits the radio signal received by the antenna unit 41a to the receiving apparatus 4 and transmits power supplied from the receiving apparatus 4 to the active circuit 41b. The receiving antennas 42 to 48 have the same configuration as that of the receiving antenna 41, and description thereof will not be provided. In the following description, when indicating any one of the respective receiving antennas 41 to 48, the receiving antenna will be described as a receiving antenna 40 (an antenna unit 40a, an active circuit 40b, and an antenna cable 40a).

The antenna changeover selection switch unit 49 is configured using a mechanical switch or a semiconductor switch, for example. The antenna changeover selection switch unit 49 is electrically connected to the respective receiving antennas 41 to 48 via a capacitor C1. When a switching signal S1 for switching the receiving antenna 40 that receives the radio signal is input from the control unit 58, the antenna changeover selection switch unit 49 selects the receiving antenna 40 indicated by the switching signal S1 and outputs the radio signal received via the selected receiving antenna 40 to the receiving circuit 50. The capacitance of the capacitor connected to each of the respective receiving antennas 41 to 48 is the same as the capacitance of the capacitor C1.

The receiving circuit 50 performs predetermined processing (for example, demodulation and amplification) on the radio signal received via the receiving antenna 40 selected by the antenna changeover selection switch unit 49 and outputs the radio signal to the signal processing circuit 51 and the received electric-field strength detector 52.

The signal processing circuit 51 extracts image data from the radio signal input from the receiving circuit 50, performs predetermined processing (for example, various types of image processing or A/D conversion) on the extracted image data, and outputs the image data to the control unit 58.

The received electric-field strength detector 52 detects received electric-field strength corresponding to the strength of the radio signal input from the receiving circuit 50 and outputs a received signal strength indicator (RSSI) corresponding to the detected received electric-field strength to the control unit 58.

The antenna power changeover selector 53 is electrically connected to the respective receiving antennas 41 to 48 via a coil L1. The antenna power changeover selector 53 supplies power to the receiving antenna 40 selected by the antenna changeover selection switch unit 49 via the antenna cable 40a. The antenna power changeover selector 53 includes a power changeover selection switch unit 531 and an abnormality detector 532. The electrical properties of the coil connected to each of the respective receiving antennas 41 to 48 are the same as the electrical properties of the coil L1.

The power changeover selection switch unit 531 is configured using a mechanical switch or a semiconductor switch, for example. When a selection signal S2 for selecting the receiving antenna 40 that supplies power is input from the control unit 58, the power changeover selection switch unit 531 selects the receiving antenna 40 indicated by the selection signal S2 and supplies power to only the selected receiving antenna 40.

When an abnormality occurs in the receiving antenna 40 to which power is supplied, the abnormality detector 532 outputs an abnormality signal indicating that an abnormality occurs in the receiving antenna 40 to which power is supplied to the control unit 58.

The display unit 54 is configured using a display panel formed of a liquid crystal or an electro luminescence (EL) material. The display unit 54 displays an image corresponding to the image data captured by the capsule endoscope 3, an operation state of the receiving apparatus 4, and various types of information such as patient information and examination date of the subject 2.

The storage unit 55 is configured using semiconductor memory such as flash memory or random access memory (RAM) that is fixedly provided inside the receiving apparatus 4. The storage unit 55 stores the image data captured by the capsule endoscope 3 and various types of information such as, for example, the position information of the capsule endoscope 3, the direction information of the capsule endoscope 3, the received electric-field strength information, or the identification information for identifying the receiving antenna that received the radio signal, correlated with the image data. The storage unit 55 stores various programs executed by the receiving apparatus 4. The storage unit 55 may include the function of a recording medium interface that stores information in a recording medium such as a memory card from the outside and reads information stored in the recording medium.

The I/F unit 56 includes the function of a communication interface and performs bidirectional communication with the image display device 5 via the cradle 5a.

The power supply unit 57 is configured using a battery that is detachable from the receiving apparatus 4 and a switch unit that switches between ON and OFF states. The power supply unit 57 supplies driving power necessary for the respective constituent units of the receiving apparatus 4 in its ON state and stops the driving power supplied to the respective constituent units of the receiving apparatus 4 in its OFF state.

The control unit 58 is configured using a central processing unit (CPU). The control unit 58 reads and executes a program from the storage unit 55 and transmits instructions and data to the respective constituent units of the receiving apparatus 4 to thereby control the operation of the receiving apparatus 4 in a centralized manner. The control unit 58 includes a selection controller 581 and an abnormality information adding unit 582.

The selection controller 581 performs control of selecting one receiving antenna 40 that receives the radio signal transmitted from the capsule endoscope 3 and supplying power to only the selected receiving antenna 40. Specifically, the selection controller 581 performs control of selecting one receiving antenna 40 that receives the radio signal transmitted from the capsule endoscope 3 based on the received electric-field strength of the respective receiving antennas 41 to 48 detected by the received electric-field strength detector 52 and supplying power to only the selected receiving antenna 40. The selection controller 581 drives the antenna changeover selection switch unit 49 every predetermined timing (for example, every 100 msec), sequentially selects the receiving antenna 40 that receives the radio signal among the respective receiving antennas 41 to 48, and causes the received electric-field strength detector 52 to detect the received electric-field strength.

When the abnormality detector 532 detects abnormality in any one of the respective receiving antennas 41 to 48, the abnormality information adding unit 582 outputs the radio signal received by the receiving antenna 40 to the storage unit 55 by adding abnormality information indicating that abnormality has occurred in any one of the respective receiving antennas 41 to 48 to the radio signal. Specifically, the abnormality information adding unit 582 outputs the image data obtained by the signal processing circuit 51 performing signal processing on the radio signal received by the receiving antenna 40 to the storage unit 55 by adding the abnormality information (flag) to the image data.

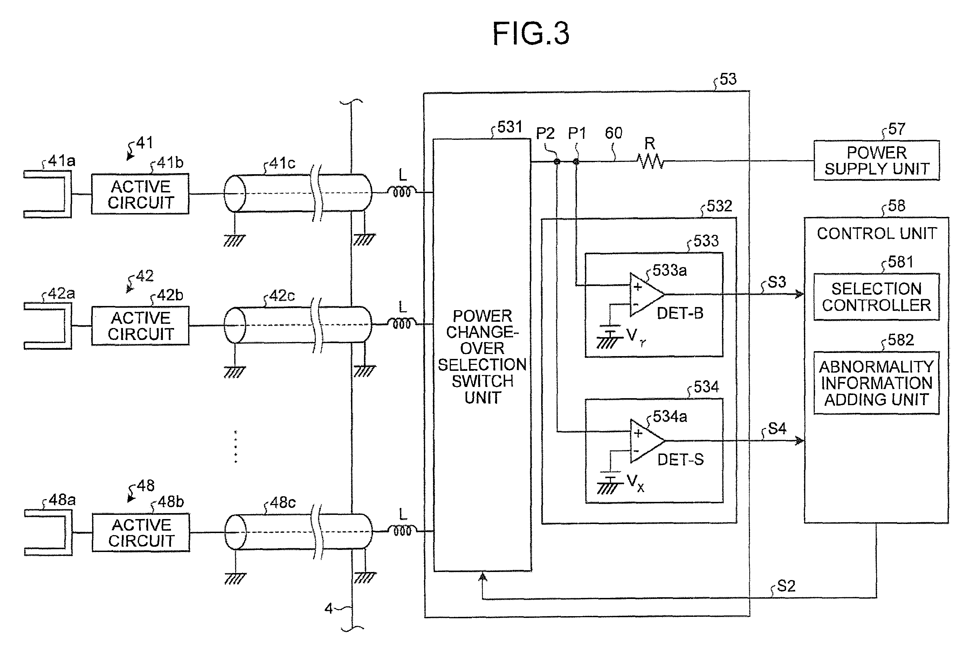

Here, the configuration of the antenna power changeover selector 53 will be described with reference to FIG. 3. FIG. 3 is a block diagram illustrating the configuration of the antenna power changeover selector 53. As illustrated in FIG. 3, the power changeover selection switch unit 531 and the abnormality detector 532 are electrically connected to the antenna power changeover selector 53.

The power changeover selection switch unit 531 has one end connected to the respective receiving antennas 41 to 48 and the other end connected to the power supply unit 57 via a detection resistor R. When the selection signal S2 for selecting the receiving antenna 41 that supplies power is input from the control unit 58, for example, the power changeover selection switch unit 531 selects only the receiving antenna 41 indicated by the selection signal S2 and electrically connects the selected receiving antenna 41 and the power supply unit 57. In this way, power is supplied to the active circuit 41b via the power changeover selection switch unit 531 and the antenna cable 41c.

The abnormality detector 532 is provided to be branched in the midway of a transmission path 60 that connects the detection resistor R and the power changeover selection switch unit 531. The abnormality detector 532 includes a disconnection abnormality detecting circuit 533 that detects disconnection abnormality of the receiving antenna 40 and a short-circuit abnormality detecting circuit 534 that detects short-circuit abnormality of the receiving antenna 40.

The disconnection abnormality detecting circuit 533 includes a comparator 533a that compares an input voltage with a threshold voltage V_{γ} and outputs the result of the comparison as a detection signal S3. The threshold voltage V_{γ} is input to one input terminal of the comparator 533a and the voltage from the transmission path 60 is input to the other input terminal. When the power changeover selection switch unit 531 selects the receiving antenna 41, and disconnection abnormality does not occur in the antenna cable 41c, since the voltage input from the transmission path 60 becomes equal to or lower than the threshold voltage V_{γ}, the comparator 533a outputs a detection signal S3_{Lo} indicating that the voltage input from the transmission path 60 is equal to or lower than the threshold voltage V_{γ} to the control unit 58. As a result, the control unit 58 determines that disconnection abnormality has not occurred in the receiving antenna 41.

On the other hand, when the power changeover selection switch unit 531 selects the receiving antenna 41, and disconnection abnormality occurs in the antenna cable 41c, since the voltage input from the transmission path 60 is higher than the threshold voltage V_{γ}, the comparator 533a outputs a detection signal S3_{Hi} indicating that the voltage input from the transmission path 60 is higher than the threshold voltage V_{γ}. As a result, the control unit 58 determines that disconnection abnormality has occurred in the receiving antenna 41. When disconnection abnormality does not occur in the receiving antenna 40, the voltage of a branch point P1 is a voltage V_{α}, and when disconnection abnormality occurs in the receiving antenna 40, since the voltage effect at the detection resistor R disappears almost completely, the voltage of the branch point P1 becomes the same voltage V_{β} as the power voltage. Thus, the threshold voltage V_{γ} is set to a voltage between the voltage V_{α} and the voltage V_{β} (V_{α} < V_{γ} < V_{β}).

The short-circuit abnormality detecting circuit 534 includes a comparator 534a that compares the input voltage with a threshold voltage V_{X} and outputs the result of the comparison as a detection signal S4. The threshold voltage V_{X} is input to one input terminal of the comparator 534a and the voltage from the transmission path 60 is input to the other input terminal. When the power changeover selection switch unit 531 selects the receiving antenna 41, and short-circuit abnormality does not occur in the antenna cable 41c, since the voltage input from the transmission path 60 is higher than the threshold voltage V_{X}, the comparator 534a outputs a detection signal S4_{Hi} indicating that the voltage input from the transmission path 60 is higher than the threshold voltage V_{X} to the control unit 58. As a result, the control unit 58 determines that short-circuit abnormality has not occurred in the receiving antenna 41.

On the other hand, when the power changeover selection switch unit 531 selects the receiving antenna 41, and short-circuit abnormality occurs in the antenna cable 41c, since the voltage input from the transmission path 60 is equal to or lower than the threshold voltage V_{X}, the comparator 534a outputs a detection signal S4_{Lo} indicating that the voltage input from the transmission path 60 is lower than the threshold voltage V_{X} to the control unit 58. As a result, the control unit 58 determines that short-circuit abnormality has occurred in the receiving antenna 41. When short-circuit abnormality occurs in the receiving antenna 40, the voltage of a branch point P2 becomes the ground voltage V_{G} (= 0). Thus, the threshold voltage V_{X} is set to a voltage between the voltage V_{α} and the voltage V_{G} (V_{G} < V_{X} < V_{α}).

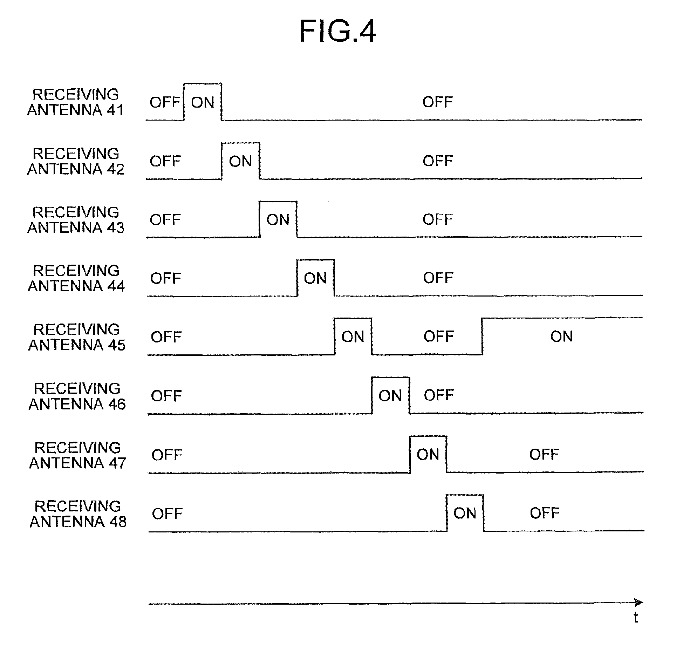

Antenna changeover processing and power changeover processing performed by the selection controller 581 in the receiving apparatus 4 configured in this manner will be described. FIG. 4 is a timing chart of the antenna changeover processing and the power changeover processing performed by the selection controller 581.

As illustrated in FIG. 4, first, in response to the startup of the receiving apparatus 4, the selection controller 581 performs control of causing the antenna changeover selection switch unit 49 to switch the receiving antennas every predetermined timing (for example, 100 msec) so as to select a receiving antenna and causing the antenna power changeover selector 53 to supply power to the selected receiving antenna. In this case, the disconnection abnormality detecting circuit 533 and the short-circuit abnormality detecting circuit 534 detect disconnection abnormality and short-circuit abnormality of the receiving antenna 40 that is sequentially switched and selected by the selection controller 581 and outputs the detection results to the control unit 58. The control unit 58 determines whether abnormality occurs in the respective receiving antennas 41 to 48 based on the detection result of each of the disconnection abnormality detecting circuit 533 and the short-circuit abnormality detecting circuit 534. The control unit 58 may output the detection results of the respective receiving antennas 41 to 48 to the display unit 54. In this way, since the user can check whether abnormality has occurred in any one of the respective receiving antennas 41 to 48, it is possible to prevent examination from being unnecessary depending on abnormality of the receiving antenna.

After preprocessing following the startup of the receiving apparatus 4, the capsule endoscope 3 is inserted into the subject 2. Following the insertion, the selection controller 581 performs control of sequentially switching and selecting the receiving antenna that receives the radio signal transmitted from the capsule endoscope 3 every predetermined timing and supplying power to only the selected receiving antenna.

Subsequently, the selection controller 581 selects a receiving antenna having the highest received electric-field strength among the respective receiving antennas 41 to 48 detected by the received electric-field strength detector 52 and supplies power to only the selected receiving antenna. In the case of FIG. 4, the selection controller 581 selects the receiving antenna 45 and supplies power to only the receiving antenna 45.

After that, the selection controller 581 performs control of switching and selecting the receiving antenna 40 that receives the radio signal transmitted from the capsule endoscope 3 every predetermined timing and supplying power to only the selected receiving antenna 40 until the capsule endoscope 3 is excreted from the subject 2. In this case, the disconnection abnormality detecting circuit 533 and the short-circuit abnormality detecting circuit 534 detect disconnection abnormality and short-circuit abnormality of the receiving antenna 40 selected by the selection controller 581 and outputs the detection results to the control unit 58. The control unit 58 determines whether abnormality has occurred in the respective receiving antennas 41 to 48 based on the detection result of each of the disconnection abnormality detecting circuit 533 and the short-circuit abnormality detecting circuit 534.

When abnormality occurs in any one of the respective receiving antennas 41 to 48, the abnormality information adding unit 582 stores the image data, which is received by any one of the receiving antennas 41 to 48 and which is processed by the signal processing circuit 51, in the storage unit 55 by adding abnormality information indicating that abnormality has occurred in any one of the receiving antennas 41 to 48 to the image data. In this way, since the image display device 5 displays the abnormality information added to the image data when the image display device 5 displays the image of the inside of the subject 2 captured by the capsule endoscope 3, the user can determine when and whether abnormality has occurred in the receiving antenna 40 and whether the image data can be used for examination.

According to the exemplary embodiment of the invention described above, the selection controller 581 performs control of selecting any one of the receiving antennas 41 to 48 as the receiving antenna that receives the radio signal transmitted from the outside and supplying power to only the selected receiving antenna 40. As a result, it is possible to suppress power consumption to the minimum even when multiple active antennas including an active circuit are used and to reduce the influence of the interference between the respective receiving antennas 41 to 48.

Further, according to the exemplary embodiment of the invention, the disconnection abnormality detecting circuit 533 and the short-circuit abnormality detecting circuit 534 detect disconnection abnormality and short-circuit abnormality of the receiving antenna 40 selected by the selection controller 581 and output the detection results to the control unit 58. As a result, the control unit 58 can easily determine whether abnormality has occurred in the receiving antenna 40 during the startup of the capsule endoscope 3 and the receiving apparatus 4 or the examination of the subject 2.

Furthermore, according to the exemplary embodiment of the invention, since the detection resistor R also serves as a current-limiting resistor, over-current due to short-circuit abnormality of the receiving antenna 40 is prevented from flowing in the receiving apparatus 4. In this way, when short-circuit abnormality occurs in the receiving apparatus 4, it is sufficient to replace only the receiving antenna 40 where short-circuit abnormality occurred while preventing the respective constituent circuits of the receiving apparatus 4 from being burnt out.

### (Other Embodiments)

In the above-described exemplary embodiment, although multiple receiving antennas including an active circuit are individually arranged at predetermined positions on the outer surface of the subject, the multiple receiving antennas including the active circuit may be arranged on one sheet-like plate.

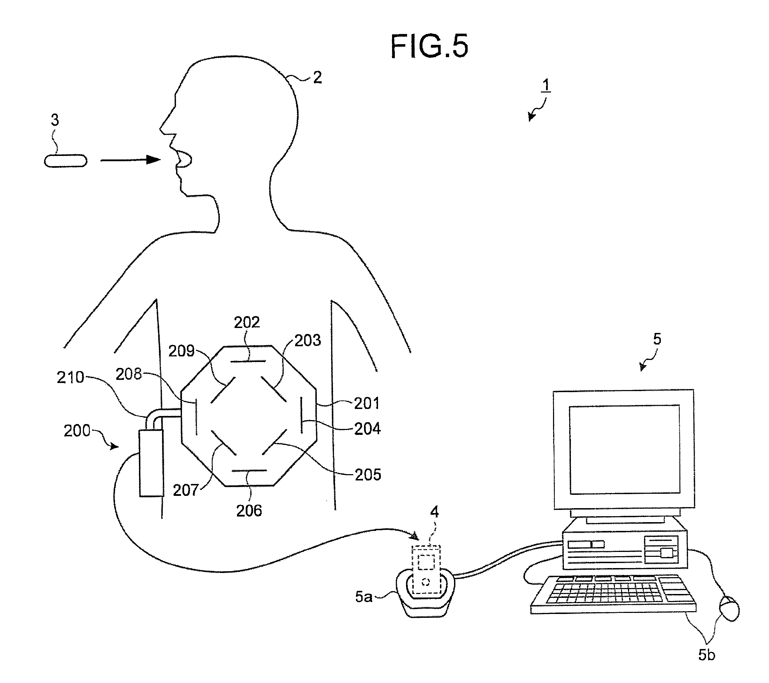

FIG. 5 is a schematic diagram illustrating the schematic configuration of a capsule endoscope system using a receiving apparatus according to a modification example of the exemplary embodiment of the invention. As illustrated in FIG. 5, a receiving apparatus 200 includes a receiving antenna unit 201. The receiving antenna unit 201 includes multiple receiving antennas 202 to 209 that form a sheet-like shape and include an active circuit and an antenna cable 210 that connects the receiving antenna unit 201 and the receiving apparatus 200.

According to the modification example of the exemplary embodiment of the invention configured in this manner, the number of antenna cables that connect the respective receiving antennas 202 to 209 and the receiving apparatus 200 is decreased. Thus, it is possible to alleviate the burden on the subject 2 and to reduce the occurrence of failure of the antenna cable. Further, since an active circuit is provided in the respective receiving antennas 202 to 209, it is possible to receive the radio signal transmitted from the capsule endoscope 3 without the need to closely attaching the respective receiving antennas 202 to 209 to the subject 2.

In the above-described exemplary embodiment, although an open-type antenna has been illustrated, the type of antenna is not particularly limited, but one in which an active circuit is provided in a loop antenna may be used, for example.

In the above-described exemplary embodiment, although the abnormality detector 532 has detected abnormality of the receiving antenna 40 based on a voltage, the abnormality of the receiving antenna 40 may be detected based on a current and/or power, for example. Further, the abnormality detector 532 may detect the abnormality of the receiving antenna 40 based on a combination of voltage, current, and power.

In the above-described exemplary embodiment, the image display device 5 can acquire the in-vivo image data captured by the capsule endoscope 2 in various methods. For example, the receiving apparatus 4 may use a memory card that is detachable from the receiving apparatus 4, such as a USB memory or a Compact Flash (registered trademark) instead of the internal storage unit 55. In this case, after the image data from the capsule endoscope 3 is stored in a memory, only the memory may be removed from the receiving apparatus 4 and inserted into a USB port or the like of the image display device 5, for example. Alternatively, the image display device 5 may have a function of communicating with an external device and acquire image data from the receiving apparatus 4 by cable or radio communication.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

### Reference Signs List

- 1: capsule endoscope system
- 2: subject
- 3: capsule endoscope
- 4, 200: receiving apparatus
- 5: image display device
- 5a: cradle
- 5b: operation input device
- 41-48, 202-209: receiving antenna
- 41a-48a: antenna unit
- 41b-48b: active circuit
- 41c-48c, 210: antenna cable
- 49: antenna changeover selection switch unit
- 50: receiving circuit
- 51: signal processing circuit
- 52: received electric-field strength detector
- 53: antenna power changeover selector
- 54: display unit
- 55: storage unit
- 56: I/F unit
- 57: power supply unit
- 58: control unit
- 60: transmission path
- 531: power changeover selection switch unit
- 532: abnormality detector
- 533: disconnection abnormality detecting circuit
- 533a, 534a: comparator
- 534: short-circuit abnormality detecting circuit
- 581: selection controller
- 582: abnormality information adding unit
- 201: receiving antenna unit

## Claims

1. A receiving apparatus comprising:
a plurality of receiving antennas in which active circuits are provided; and
a selection controller that performs control of selecting one receiving antenna that receives a radio signal transmitted from the outside from the plurality of receiving antennas and supplying power only to the selected receiving antenna.

2. The receiving apparatus according to claim 1, further comprising an abnormality detector that detects abnormality of the selected receiving antenna based on a voltage when power is supplied to the receiving antenna selected by the selection controller.

3. The receiving apparatus according to claim 2, wherein
the abnormality is disconnection abnormality or short-circuit abnormality of the selected receiving antenna.

4. The receiving apparatus according to claim 2, further comprising an abnormality information adding unit that adds abnormality information indicating that abnormality has occurred in the receiving antenna to the radio signal received by the receiving antenna when the abnormality detector detects abnormality of the receiving antenna.

5. The receiving apparatus according to claim 1, further comprising a received electric-field strength detector that detects received electric-field strength of an electric field received by each of the plurality of receiving antennas, wherein
the selection controller selects the receiving antenna that receives the radio signal based on the received electric-field strength detected by the received electric-field strength detector.

6. The receiving apparatus according to claim 1, further comprising:
an antenna selection changeover switch unit that selects one antenna cable from a plurality of antenna cables respectively connected to the plurality of receiving antennas and performs switching of the connection; and
an antenna power changeover selector that supplies power to the antenna cable selected from the plurality of antenna cables respectively connected to the plurality of receiving antennas, wherein
the selection controller drives the switching operation of the antenna selection changeover switch unit and the antenna power changeover selector in a synchronized manner.

7. A capsule endoscope system comprising:
a capsule endoscope that is inserted into a subject and transmits image data of the inside of the subject to the outside via a radio signal; and
a receiving apparatus including
a plurality of receiving antennas in which an active circuit is provided, and
a selection controller that performs control of selecting one receiving antenna that receives a radio signal transmitted from the capsule endoscope from the plurality of receiving antennas and supplying power only to the selected receiving antenna.

8. The capsule endoscope system according to claim 7, the receiving apparatus further includes an abnormality detector that detects abnormality of the selected receiving antenna based on a voltage when power is supplied to the receiving antenna selected by the selection controller.

9. The capsule endoscope system according to claim 8, wherein
the abnormality is disconnection abnormality or short-circuit abnormality of the selected receiving antenna.

10. The capsule endoscope system according to claim 8, wherein
the receiving apparatus further includes an abnormality information adding unit that adds abnormality information indicating that abnormality has occurred in the receiving antenna to the radio signal received by the receiving antenna when the abnormality detector detects abnormality of the receiving antenna.

11. The capsule endoscope system according to claim 7, wherein
the receiving apparatus further includes a received electric-field strength detector that detects received electric-field strength of an electric field received by each of the plurality of receiving antennas, and
the selection controller selects the receiving antenna that receives the radio signal based on the received electric-field strength detected by the received electric-field strength detector.

12. The capsule endoscope system according to claim 7, wherein
the receiving apparatus further includes:
an antenna selection changeover switch unit that selects one antenna cable from a plurality of antenna cables respectively connected to the plurality of receiving antennas and performs switching of the connection; and
an antenna power changeover selector that supplies power to the antenna cable selected from the plurality of antenna cables respectively connected to the plurality of receiving antennas, and
the selection controller drives the switching operation of the antenna selection changeover switch unit and the antenna power changeover selector in a synchronized manner.
